# EUROPEAN PATENT APPLICATION

(11) **EP 1 695 719 A1**
(43) Date of publication of application: **30.08.2006**
(21) Application number: 05026675.8
(22) Date of filing: 09.02.1999
(51) Int. Cl.: A61K 48/00

(54) **Combination of a nucleic acid and a vasoactive agent for enhanced gene delivery**

(30) Priority: 11.02.1998 US 21773
(62) Divisional of application: 99906814.1
(71) Applicant: The Regents of the University of California, Oakland, CA 94607 (US)
(72) Inventor: Hammond, Kirk H., La Jolla, CA 92037 (US)
(74) Representative: Campbell, Patrick John Henry

(57) **Abstract**

Transgene-inserted vectors are effectively used for *in vivo* gene therapy for peripheral vascular disease, heart disease and other conditions, by direct injection of the vector into arteries supplying the tissue to be targeted, preferably in combination with a vasoactive agent that is infused into the artery prior to or coincident with delivery of the vector.

### Annex to the application documents - subsequently filed sequences listing

SEQUENCE LISTING

<110> The Regents of the University of California

<120> Combination of a nucleic acid and a vasoactive agent for enhanced
gene delivery

<130> N.77781C PJC

<140> EP 05026675.8
<141> 1999-02-09

<150> EP 99906814.1
<151> 1999-02-09

<150> PCT/US99/02702
<151> 1999-02-09

<150> US 09/021,773
<151> 1998-02-11

<160> 3

<170> PatentIn version 3.2

<210> 1
<211> 20
<212> DNA
<213> Artificial sequence

<220>
<223> primer

<400> 1
gcagagctcg tttagtgaac 20

<210> 2
<211> 21
<212> DNA
<213> Artificial sequence

<220>
<223> primer

<400> 2
gaaaatgggt agagatatgc t 21

<210> 3
<211> 21
<212> DNA
<213> Artificial sequence

<220>
<223> primer

<400> 3
atgagcttgt ccttcctcct c 21

## Description

### BACKGROUND

### Field of the Invention

The present invention relates to gene therapy, more specifically, to virus-mediated and other forms of gene therapy, and to certain vector constructs, including adenovirus constructs, and techniques useful in the delivery of desired genes. More particularly, the invention relates to vector-mediated delivery of genes useful in the promotion of angiogenesis in the heart, and to methods for the treatment of peripheral vascular disease and diseases of the heart, including myocardial ischemia, and other diseases using such vectors and gene delivery techniques.

### Background of the Art

It has been reported by the American Heart Association (1995 Statistical Supplement), that there are about 60 million adults in the United States that have cardiovascular disease, including 11 million adults who have coronary heart disease. Cardiovascular diseases are responsible for almost a million deaths annually in the United States representing over 40% of all deaths. In 1995, 1.5 million adults in the United States will carry the diagnosis of angina pectoris, experiencing transient periods of myocardial ischemia resulting in chest pain. About 350,000 new cases of angina occur each year in the United States.

Myocardial ischemia occurs when the heart muscle does not receive an adequate blood supply and is thus deprived of necessary levels of oxygen and nutrients. The most common cause of myocardial ischemia is atherosclerosis, which causes blockages in the blood vessels (coronary arteries) that provide blood flow to the heart muscle. Present treatments include pharmacological therapies, coronary artery bypass surgery and percutaneous revascularization using techniques such as balloon angioplasty. Standard pharmacological therapy is predicated on strategies that involve either increasing blood supply to the heart muscle or decreasing the demand of the heart muscle for oxygen and nutrients. Increased blood supply to the myocardium is achieved by agents such as calcium channel blockers or nitroglycerin. These agents are thought to increase the diameter of diseased arteries by causing relaxation of the smooth muscle in the arterial walls. Decreased demand of the heart muscle for oxygen and nutrients is accomplished either by agents that decrease the hemodynamic load on the heart, such as arterial vasodilators, or those that decrease the contractile response of the heart to a given hemodynamic load, such as beta-adrenergic receptor antagonists. Surgical treatment of ischemic heart disease is based on the bypass of diseased arterial segments with strategically placed bypass grafts (usually saphenous vein or internal mammary artery grafts). Percutaneous revascularization is based on the use of catheters to reduce the narrowing in diseased coronary arteries. All of these strategies are used to decrease the number of, or to eradicate, ischemic episodes, but all have various limitations.

Preliminary reports describe new vessel development in the heart through the direct injection of angiogenic proteins or peptides to treat myocardial ischemia. The several members of the fibroblast growth factor (FGF) family (namely acidic fibroblast growth factor, aFGF; basic fibroblast growth factor, bFGF; fibroblast growth factor-5, FGF-5 and others) have been implicated in the regulation of angiogenesis during growth and development. The role of aFGF protein in promoting angiogenesis in adult animals, for example, was the subject of a recent report. It states that aFGF protein, within a collagen-coated matrix, placed in the peritoneal cavity of adult rats, resulted in a well vascularized and normally perfused structure (Thompson, et al., *PNAS* 86:7928-7932, 1989). Injection of bFGF protein into adult canine coronary arteries during coronary occlusion reportedly led to decreased myocardial dysfunction, smaller myocardial infarctions, and increased vascularity in the bed at risk (Yanagisawa-Miwa, et al., *Science* 257:1401-1403, 1992). Similar results have been reported in animal models of myocardial ischemia using bFGF protein (Harada, et al., *J Clin Invest* 94:623-630, 1994. Unger, et al., *Am J Physiol* 266:H1588-H1595, 1994).

A prerequisite for achieving an angiogenic effect with these proteins however, has been the need for repeated or long term delivery of the protein, which limits the utility of using these proteins to stimulate angiogenesis in clinical settings. In other words, successful therapy in humans would require sustained and long-term infusion of one or more of these angiogenic peptides or proteins, which are themselves prohibitively expensive and which would need to be delivered by catheters placed in the coronary arteries, further increasing the expense and difficulty of treatment.

Recently, various publications have postulated on the uses of gene transfer for the treatment or prevention of disease, including heart disease. *See, for example,* Mazur et al., "Coronary Restenosis and Gene Therapy," *Molecular and Cellular Pharmacology,* 21:104-111, 1994; French, "Gene Transfer and Cardiovascular Disorders," *Herz* 18:222-229, 1993; Williams, "Prospects for Gene Therapy of lschemic Heart Disease," *American Journal of Medical Sciences* 306:129-136, 1993; Schneider and French, "The Advent of Adenovirus: Gene Therapy for Cardiovascular Disease," *Circulation* 88:1937-1942, 1993. Another publication, Leiden et al, International Patent Application Number PCT/US93/11133, entitled "Adenovirus-Mediated Gene Transfer to Cardiac and Vascular Smooth Muscle," reports on the use of adenovirus-mediated gene transfer for the purpose of regulating function in cardiac vascular smooth muscle cells. Leiden et al. states that a recombinant adenovirus comprising a DNA sequence that encodes a gene product can be delivered to a cardiac or vascular smooth muscle cell and the cell maintained until that gene product is expressed. According to Leiden et al., muscle cell function is regulated by altering the transcription of genes and changes in the production of a gene transcription product, such as polynucleotide or polypeptide.

There are impediments to successful gene transfer to organs such as the heart using adenovirus and other vectors. For example, the insertion of a transgene into a rapidly dividing cell population will result in substantially reduced duration of transgene expression. Examples of such cells include endothelial cells, which make up the inner layer of all blood vessels, and fibroblasts which are dispersed throughout the heart. Targeting the transgene so that only the desired cells will receive and express the transgene, and the transgene will not be systemically distributed, are also critically important considerations. If this is not accomplished, systemic expression of the transgene and problems attendant thereto will result. For example, inflammatory infiltrates have been documented after adenovirus-mediated gene transfer in liver (Yang, et al. *Proc. Natl*. *Acad*. *Sci.* (U.S.A.) 91:4407, 1994).

The invention described and claimed herein addresses and overcomes these and other problems associated with the prior art.

### BRIEF DESCRIPTION OF THE FIGURES

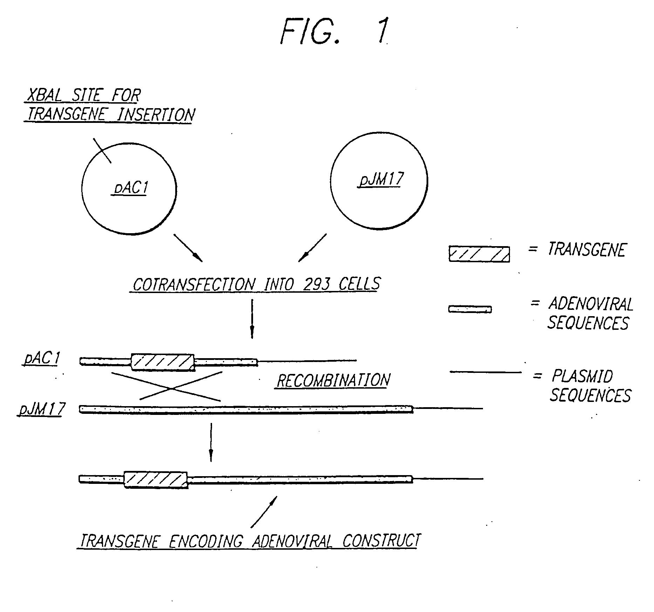

FIGURE 1 is a schematic figure which shows rescue recombination construction of a transgene encoding adenovirus.

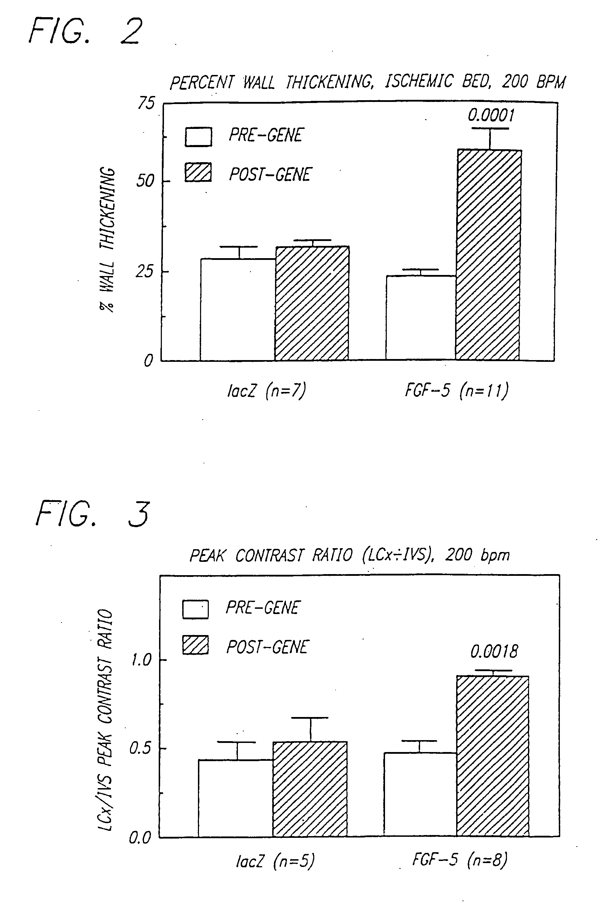

FIGURE 2 shows percent wall thickening (%WTh) in the ischemic bed during right atrial pacing (HR=200 bpm), calculated by measuring end-diastolic wall thickness (EDWTh) and end-systolic wall thickness (ESWTh) before and 14 ± 1 days after gene transfer with lacZ (control gene) and with FGF-5. Function in the ischemic bed was increased 2.6-fold after transfer with FGF-5 (p= 0.0001) but unaffected by the control gene.
FIGURE 3 shows the peak contrast ratio (a correlate of blood flow) expressed as the ratio of the peak video intensity in the ischemic region (LCx bed) divided by the peak video intensity in the interventricular septum (IVS), measured from the video images using a computer-based video analysis program during atrial pacing (200 bpm) before and 14 ± 1 days after gene transfer with lacZ (control gene) and with FGF-5. Blood flow to the ischemic bed increased 2-fold normal after gene transfer with FGF-5 (p=0.0018), but remained 50% of normal after the control gene.

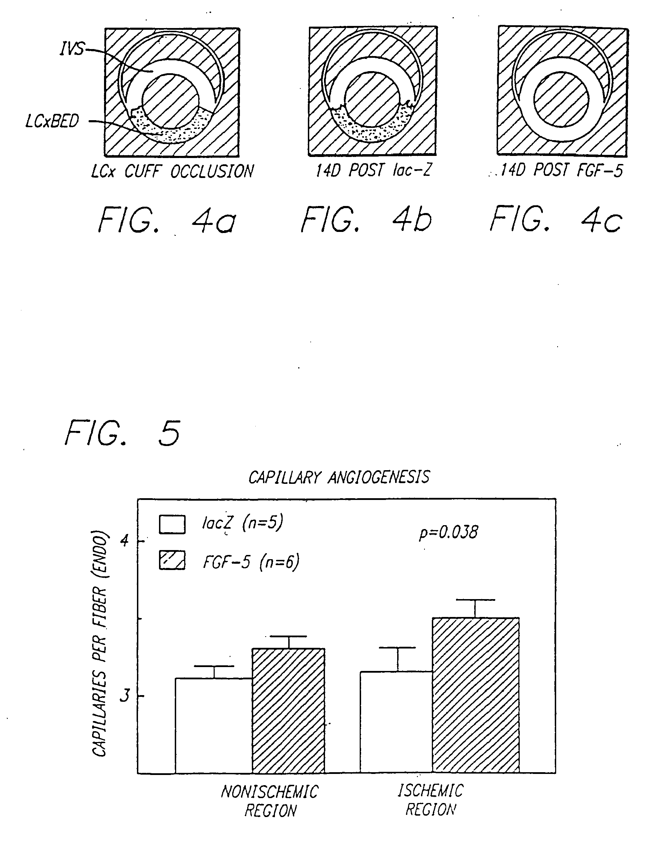

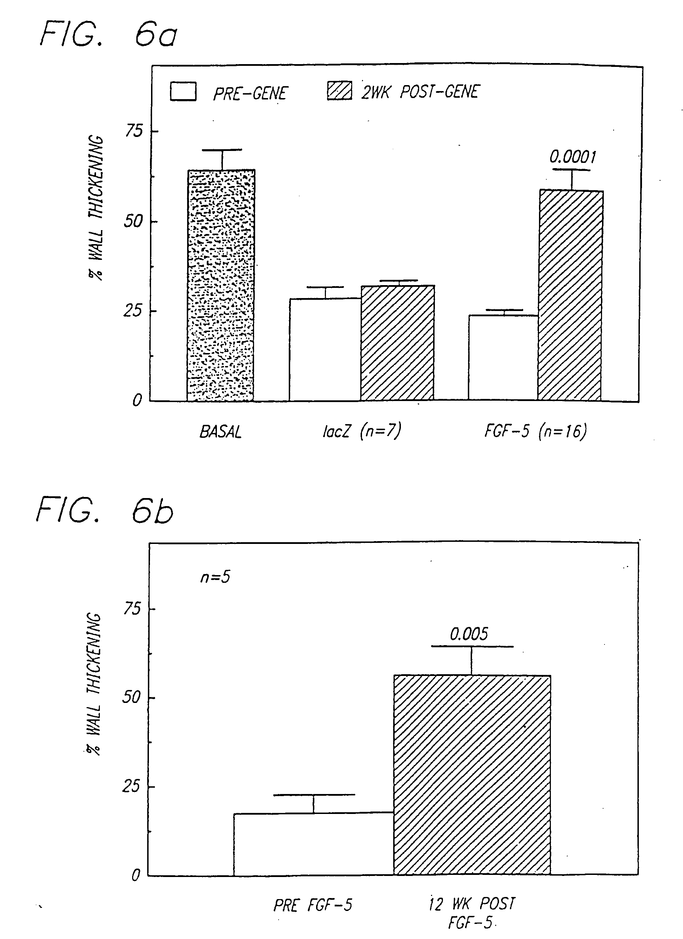

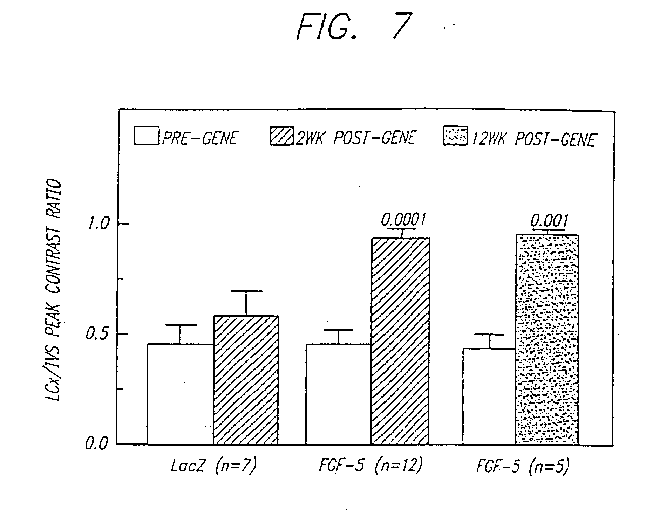

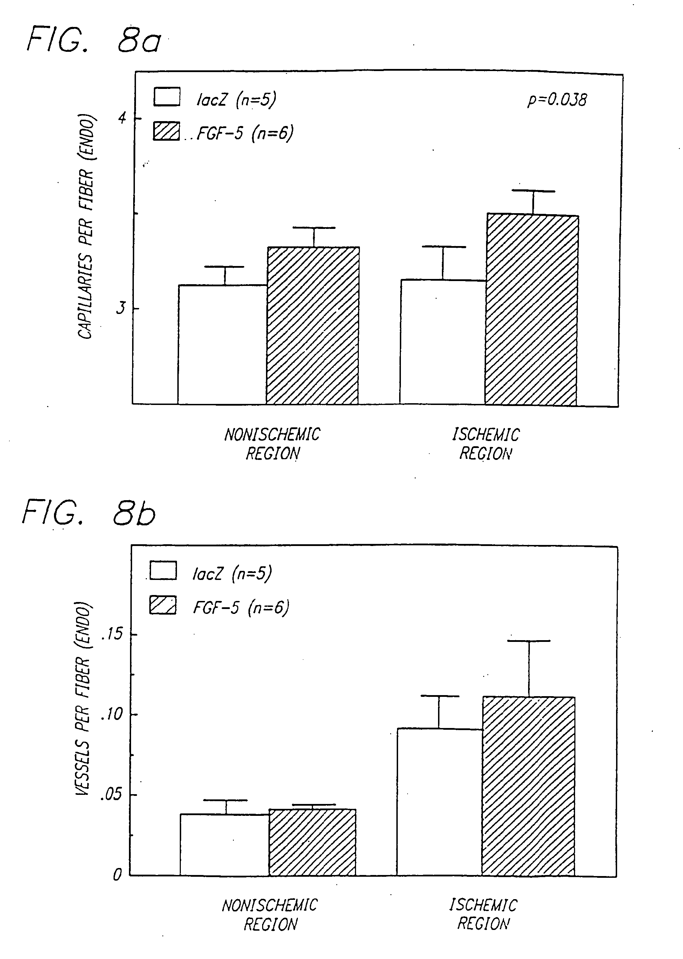

FIGURE 4 shows diagrams corresponding to myocardial contrast echocardiographs (not shown). White areas denote contrast enhancement (more blood flow) and dark areas denote decreased blood flow. FIGURE 4A shows acute LCx occlusion in a normal pig, FIGURE 4B shows 14 ± 1 days after lacZ gene transfer, and FIGURE 4C shows 14 ± 1 days after gene transfer with FGF-5.
FIGURE 5 shows the ratio of capillary number to fiber number quantitated by microscopic analysis in the ischemic and nonischemic regions after gene transfer with FGF-5 and with lacZ. There was increased angiogenesis after FGF-5 gene transfer (p<0.038).

### SUMMARY OF THE INVENTION

The present invention is directed to a gene therapy approach useful in the treatment of heart disease, such as myocardial ischemia, and peripheral vascular disease, as well as other conditions in which a gene therapy vector is introduced by intravascular delivery. One objective of the present invention is to provide a method for treating heart disease in which an angiogenic protein or peptide (such as FGF-5 or FGF-4), is produced to a therapeutically significant degree in the myocardium continuously for sustained periods by targeting the heart with a vector construct containing a gene for said angiogenic protein or peptide, preferably a viral vector such as a replication-deficient adenovirus construct, delivered through intracoronary injection, preferably by catheter introduced substantially (typically at least about 1 cm) beyond the ostium of one or both coronary arteries or one or more saphenous vein or internal mammary artery grafts. Thus, injection into one coronary artery or graft artery is contemplated. Injection into both the right and left coronary circulation is preferred. Delivery by three injections, one each into the left anterior descending (LAD) and left circumflex (LCx) coronary artery, and one into the right coronary artery, is particularly preferred.

In order to further enhance gene delivery, as described herein, the gene delivery vector is preferably administered in conjunction with infusion of a vasoactive agent (for example, histamine, a histamine agonist, or a vascular endothelial growth factor (VEGF) protein) at the site being targeted by intravascular delivery; most preferably by infusion of the vasoactive agent within several minutes prior to introduction of the vector.

Another aspect of the present invention is a method for treating a heart disease in a patient having myocardial ischemia, comprising delivering a transgene-inserted vector to the myocardium of the patient by intracoronary injection, preferably by injecting the vector directly into one or both coronary arteries (or grafts), to transfect cardiac myocytes in the affected myocardium, said vector comprising a transgene coding for an angiogenic protein or peptide, for example, FGF-5, FGF-4, aFGF, bFGF or VEGF (vascular endothelial growth factor), and expressing the transgene in the heart, thereby promoting angiogenesis in the affected region of the myocardium. Other transgenes, such as those encoding β-adrenergic signaling proteins, can also be used, as described below. The vectors employed in the invention can be a plasmid or preferably a viral vector, for example a replication-deficient adenovirus or adeno-associated virus (AAV). By injecting the viral vector stock such as one that contains no wild-type virus deeply into the lumen of one or both coronary arteries (or grafts), preferably into both the right and left coronary arteries (or grafts), and preferably in an amount of 10⁷-10¹³ viral particles as determined by optical densitometry (more preferably 10⁹-10¹¹ viral particles), it is possible to locally transfect a desired number of cells, especially cardiac myocytes, in the affected myocardium with angiogenic protein-or peptide-encoding genes, thereby maximizing therapeutic efficacy of gene transfer, and minimizing undesirable angiogenesis at extracardiac sites and the possibility of an inflammatory response to viral proteins. If a ventricular myocyte-specific promoter is used, for example, the promoter more securely enables expression limited to the cardiac myocytes so as to avoid the potentially harmful effects of angiogenesis in non-cardiac tissues such as the retina. To further enhance the localized delivery of the gene delivery vector in accordance with the inventions, one can infuse a vasoactive agent, preferably histamine or a histamine agonist or a vascular endothelial growth factor (VEGF) protein, to the myocardium utilizing a catheter in a manner as described herein.

In another aspect, the present invention provides a filtered injectable adenoviral vector preparation, comprising a recombinant adenoviral vector, preferably in a final viral titer of 10⁷-10¹³ viral particles, said vector containing no wild-type virus and comprising a partial adenoviral sequence from which one or more required adenovirus genes conferring replication competence, for example, the ElA/ElB genes have been deleted, and a transgene coding for an angiogenic protein or peptide, for example aFGF, bFGF, FGF-4, FGF-5 and VEGF, driven by a promoter flanked by the partial adenoviral sequence; and a pharmaceutically acceptable carrier. By using this injectable adenoviral vector preparation, optionally in combination with a vasoactive agent, for example histamine or a histamine agonist or a vascular endothelial growth factor (VEGF) protein, to enhance gene delivery as described herein, it is possible to perform effective adenovirus-mediated FGF gene transfer for the treatment of heart disease, for example clinical myocardial ischemia, or peripheral vascular disease without any undesirable effects.

In a further aspect, the present invention provides a method of production of a viral stock containing a recombinant vector capable of expressing an angiogenic protein or peptide *in vivo* in the myocardium, comprising the steps of cloning a transgene, preferably coding for an angiogenic protein or peptide such as FGF-4, FGF-5, aFGF, bFGF and VEGF, into a plasmid containing a promoter and a polylinker flanked by partial adenoviral sequences of the left end of the human adenovirus 5 genome from which one or more required adenovirus genes conferring replication competence, for example, the EIA/EIB genes have been deleted; co-transfecting said plasmid into mammalian cells transformed with the missing replication-requiring genes, with a plasmid which contains the entire human adenoviral 5 genome and an additional insert making the plasmid too large to be encapsulated, whereby rescue recombination takes place between the transgene-inserted plasmid and the plasmid having the entire adenoviral genome so as to create a recombinant genome containing the transgene without the replication-requiring genes, said recombinant genome being sufficiently small to be encapsulated; identifying successful recombinants in cell cultures; propagating the resulting recombinant in mammalian cells transformed with the absent replication-requiring genes; and purifying the propagated recombinants so as to contain the recombinant vector, without wild-type virus therein, and optionally passing the purified vector through a filter, preferably 10-50 micron filter, more preferably a 30 micron filter.

In yet another aspect, a recombinant adenovirus expressing an angiogenic peptide or protein will be delivered by catheter into the proximal portion of the femoral artery or arteries, preferably in combination with a vasoactive agent, for example histamine or a histamine agonist as described herein, thereby effecting gene transfer into the cells of the skeletal muscles receiving blood flow from the femoral arteries. This will provide an angiogenic stimulus that will result in angiogenesis in skeletal muscle of the legs and will serve as a treatment for peripheral vascular disease, a disease that is characterized by insufficient blood supply to muscles of the legs.

In a further aspect of the present invention, the efficiency of gene delivery using a vector such as a viral vector (e.g. adenovirus or adeno-associated virus) is enhanced by delivering the vector into a blood vessel such as an artery that is co-infused or pre-infused with a vasoactive agent, for example histamine or a histamine agonist, or a vascular endothelial growth factor (VEGF) protein, as described herein. Most preferably the vasoactive agent is infused into the blood vessel within several minutes prior to introduction of the vector.

### DETAILED DESCRIPTION OF THE PREFERRED EMBODIMENTS

### Transgenes of the Present Invention

In the present invention, various protein or peptide growth factors that are capable of improving myocardial blood flow to ischemic regions of the heart (or skeletal muscle in the case of peripheral vascular disease), as well as other transgenes, can be used. As an angiogenic protein or peptide to be expressed, an angiogenic protein or peptide such as aFGF, bFGF, FGF-4 and FGF-5 can be exemplified. The angiogenic activity of the FGF family is reasonably well established in the setting of protein infusions (Yanagisawa-Miwa, et al., *Science* 257:1401-1403, 1992, Harada, et al., *J Clin Invest* 94:623-630, 1994, Unger, et al., *Am J Physiol* 266:H1588-H1595, 1994). The gene for VEGF (vascular endothelial growth factor), a potent stimulator of new vessel growth, can also be used. Success of the gene transfer approach requires both synthesis of the gene product and secretion from the transfected cell. From this point of view, a gene encoding FGF-5 or FGF-4 is preferred and is preferably selected to include a sequence encoding a signal peptide, thus directing that the gene product, once expressed, will gain access to the cardiac (or skeletal muscle) interstitium and induce angiogenesis. Angiogenic proteins that can be employed thus include members of the family of fibroblast growth factors (FGF), vascular endothelial growth factors (VEGF), platelet-derived growth factors (PDGF), insulin-like growth factors (IGF), and others. Members of the FGF family include, but are not limited to, aFGF (FGF-1), bFGF (FGF-2), FGF-4 (also known as "hst/KS3"), FGF-5, FGF-6. VEGF has been shown to be expressed by cardiac myocytes in response to ischemia *in vitro* and *in vivo*; it is a regulator of angiogenesis under physiological conditions as well as during the adaptive response to pathological states ((Banai et al. Circulation 89:2183-2189, 1994). The VEGF family, includes, but is not limited to, members of the VEGF-A sub-family (e.g. VEGF-121, VEGF-145. VEGF-165, VEGF-189 and VEGF-206), as well as members of the VEGF-B sub-family (e.g. VEGF-167 and VEGF-186) and the VEGF-C sub-family. PDGF includes PDGF A and PDGF B. The nucleotide sequences of the genes encoding these proteins, and the corresponding amino acid sequences are known in the art (see. e.g., the GENBANK sequence database for these and other sequences). For the FGF family, see. e.g., Burgess, Ann. N.Y. Acad. Sci. 638: 89-97, 1991; Burgess et al. Annu. Rev. Biochem 58:575-606, 1989; Muhlhauser et al. Hum. Gene Therapy 6:1457-1465, 1995; Zhan et al., Mol. Cell. Biol., 8:3487, 1988; Seddon et al. Ann. N.Y. Acad. Sci. 638:98-108, 1991. For human hst/KS3 (i.e. FGF-4), see Taira et al. Proc. Natl. Acad. Sci. USA 84:2980-2984, 1987. For human VEGF, see e.g., Tischer et al. J. Biol. Chem. 206:11947-11954, 1991, and references therein; Muhlhauser et al., Cir. Res. 77:1077-1086, 1995). Sequence information for such genes and encoded polypeptides is readily obtainable from sequence databases such as GenBank or EMBL. Polynucleotides encoding these proteins can also be obtained from gene libraries, e.g., by using PCR or hybridization techniques routine in the art. Genes encoding FGF-4, FGF-5, or FGF-6 are preferred since these proteins contain functional secretory signal sequences and are readily secreted from cells. Many if not most human VEGF proteins (including but not limited to VEGF-121 and VEGF-165) also are readily secreted and diffusable after secretion. Thus, when expressed, these angiogenic proteins can readily access the cardiac interstitium and induce angiogenesis. With other angiogenic proteins such as aFGF (FGF-1) and bFGF (FGF-2) that lack a native secretory signal sequence, fusion proteins having secretory signal sequences can be recombinantly produced using standard recombinant DNA methodology familiar to one of skill in the art. It is believed that both aFGF and bFGF are naturally secreted to some degree; however, inclusion of an additional secretion signal sequence can be used to enhance secretion of the protein. The secretory signal sequence would typically be positioned at the N-terminus of the desired protein but can be placed at any position suitable to allow secretion of the angiogenic factor. For example, a polynucleotide containing a suitable signal sequence can be fused 5' to the first codon of the selected angiogenic protein gene. Suitable secretory signal sequences include signal sequences of the FGF-4, FGF-5, FGF-6 genes or a signal sequence of a different secreted protein such as IL-1β. Angiogenic proteins can be modified to contain a signal sequence from another protein, for example by replacement of residues in the angiogenic protein with residues that direct secretion of the secreted second protein, as described in co-pending application U.S. Serial No. 08/852,779, filed May 6, 1997, incorporated by reference herein. A signal sequence derived from a protein that is normally secreted from cardiac myocytes can be used. For treating humans, genes encoding angiogenic proteins of human origin are preferred although angiogenic proteins of other mammalian origin, that exhibit cross-species activity i.e. having angiogenic activity in humans, can also be used.

In addition to angiogenic factors, β-adrenergic signaling proteins (β-ASPs) that enhance cardiac functions can also be employed. Examples of such β-ASPs include β-adrenergic receptors (β-ARs), G-protein receptor kinase inhibitors (GRK inhibitors) and adenylylcyclases (ACs), as described in detail in co-pending applications U.S. Serial No. 08/924,757, filed 05 September 1997 (based on U.S. 60/048,933 filed 16 June 1997 and U.S. 08/708,661 filed 05 September 1996), as well as PCT/US97/15610 filed 05 September 1997, and U.S. continuing case Serial No. 08/__, filed 16 January 1998. These and all other patent applications and references cited in this patent application are incorporated by reference herein.

### Vectors for Gene Delivery

In methods of the present invention, the vector can be a plasmid vector or a viral vector, for example a replication-deficient adenoviral vector or adeno-associated viral vector. References describing the construction and use of a variety of vectors, including both viral and non-viral vectors, are provided below.

By way of illustration, a gene of interest is transferred to the heart (or skeletal muscle), including cardiac myocytes (and skeletal myocytes), *in vivo* and directs constitutive production of the encoded protein. Several different gene transfer approaches are feasible. Preferred is the helper-independent replication deficient human adenovirus system. Using this system, we have demonstrated transfection greater than 60% of myocardial cells *in vivo* by a single intracoronary injection (Giordano and Hammond. *Clin. Res*. 42: 123A, 1994). Non-replicative recombinant adenoviral vectors are particularly useful in transfecting coronary endothelium and cardiac myocytes resulting in highly efficient transfection after intracoronary injection. The same will be true for transfecting desired cells of the peripheral vascular system.

The recombinant adenoviral vectors based on the human adenovirus 5 *(Virology* 163:614-617, 1988) are missing essential early genes from the adenoviral genome (usually E1A/E1B), and are therefore unable to replicate unless grown in permissive cell lines that provide the missing gene products *in trans.* In place of the missing adenoviral genomic sequences, a transgene of interest can be cloned and expressed in tissue/cells infected with the replication deficient adenovirus. Although adenovirus-based gene transfer does not result in integration of the transgene into the host genome (less than 0.1 % adenovirus-mediated transfections result in transgene incorporation into host DNA), and therefore is not stable, adenoviral vectors can be propagated in high titer and transfect non-replicating cells. Although the transgene is not passed to daughter cells, this is acceptable for gene transfer to adult skeletal muscle and cardiac myocytes, which do not divide. Retroviral vectors provide stable gene transfer, and high titers are now obtainable via retrovirus pseudotyping (Bums, et al., *Proc Natl Acad Sci (USA)* 90:8033-8037, 1993), but current retroviral vectors are unable to transduce nonreplicating cells (adult skeletal muscle and cardiac myocytes) efficiently. In addition, the potential hazards of transgene incorporation into host DNA are not warranted if short-term gene transfer is sufficient. Indeed, we have discovered that a limited duration expression of an angiogenic protein is sufficient for substantial angiogenesis, and transient gene transfer for cardiovascular disease and peripheral disease processes is therapeutically adequate.

Human 293 cells, which are human embryonic kidney cells transformed with adenovirus E1A/E1B genes, typify useful permissive cell lines. However, other cell lines which allow replication-deficient adenoviral vectors to propagate therein can be used, including HeLa cells.

### Construction of Recombinant Adenoviral Vectors and Other Vectors for Gene Delivery

All adenoviral vectors used in the present invention can be constructed by the rescue recombination technique described in Graham, *Virology* 163:614-617, 1988. Briefly, the transgene of interest is cloned into a shuttle vector that contains a promoter, polylinker and partial flanking adenoviral sequences from which ElA/ElB genes have been deleted. As the shuttle vector, plasmid pAC 1 *(Virology* 163:614-617, 1988) (or an analog) which encodes portions of the left end of the human adenovirus 5 genome *(Virology* 163:614-617. 1988) minus the early protein encoding ElA and El B sequences that are essential for viral replication, and plasmid ACCMVPLPA *(J Biol Chem* 267:25129-25134, 1992) which contains polylinker, the CMV promoter and SV40 polyadenylation signal flanked by partial adenoviral sequences from which the E1A/E1B genes have been deleted can be exemplified. The use of plasmid pACl or ACCMVPLA facilitates the cloning process. The shuttle vector is then co-transfected with a plasmid which contains the entire human adenoviral 5 genome with a length too large to be encapsulated, into 293 cells. Co-transfection can be conducted by calcium phosphate precipitation or lipofection *(Biotechniques* 15:868-872, 1993). Plasmid JM17 encodes the entire human adenovirus 5 genome plus portions of the vector pBR322 including the gene for ampicillin resistance (4.3 kb). Although JM17 encodes all of the adenoviral proteins necessary to make mature viral particles, it is too large to be encapsulated (40 kb versus 36 kb for wild type). In a small subset of co-transfected cells, rescue recombination between the transgene containing the shuttle vector such as plasmid pAC 1 and the plasmid having the entire adenoviral 5 genome such as plasmid pJM 17 provides a recombinant genome that is deficient in the E1A/E1B sequences, and that contains the transgene of interest but secondarily loses the additional sequence such as the pBR322 sequences during recombination, thereby being small enough to be encapsulated (see Figure 1). With respect to the above method, we have reported successful results (Giordano, et al. *Circulation* 88:1-139, 1993, and Giordano and Hammond, *Clin Res* 42:123A, 1994). The CMV driven β-galactosidase encoding adenovirus HCMVSP1lacZ *(CLIN RES* 42:123A, 1994) can be used to evaluate efficiency of gene transfer using X-gal treatment.

The initial mode of gene transfer uses adenoviral vectors as delineated above. The advantages of these vectors include the ability to effect high efficiency gene transfer (more than 60% of target organ cells transfected in *vivo),* the ease of obtaining high titer viral stocks and the ability of these vectors to effect gene transfer into cells such as cardiac myocytes which do not divide.

References describing a variety of other gene delivery vectors are known in the art, some of which are cited herein. Such other vectors include, for example, other viral vectors (such as adeno-associated viruses (AAV)), liposomes and other lipid-containing complexes, and other macromolecular complexes capable of mediating delivery of a polynucleotide to a host cell. As described above and in the cited references, vectors can also comprise other components or functionalities that further modulate gene delivery and/or gene expression, or that otherwise provide beneficial properties to the targeted cells. Such other components include, for example, components that influence binding or targeting to cells (including components that mediate cell-type or tissue-specific binding); components that influence uptake of the vector nucleic acid by the cell; components that influence localization of the polynucleotide within the cell after uptake (such as agents mediating nuclear localization); and components that influence expression of the polynucleotide. Such components also might include markers, such as detectable and/or selectable markers that can be used to detect or select for cells that have taken up and are expressing the nucleic acid delivered by the vector. Such components can be provided as a natural feature of the vector (such as the use of certain viral vectors which have components or functionalities mediating binding and uptake), or vectors can be modified to provide such functionalities. Selectable markers can be positive, negative or bifunctional. Positive selectable markers allow selection for cells carrying the marker, whereas negative selectable markers allow cells carrying the marker to be selectively eliminated. A variety of such marker genes have been described, including bifunctional (i.e. positive/negative) markers (see, e.g., Lupton, S., WO 92/08796, published 29 May 1992; and Lupton, S., WO 94/28143, published 8 December 1994). Such marker genes can provide an added measure of control that can be advantageous in gene therapy contexts. A large variety of such vectors are known in the art and are generally available (see. e.g., the various references cited above).

Additional references describing adenovirus vectors and other viral vectors which could be used in the methods of the present invention include the following: Horwitz, M.S., Adenoviridae and Their Replication, in Fields, B., et al. (eds.) Virology, Vol. 2, Raven Press New York, pp. 1679-1721, 1990); Graham, F., et al., pp. 109-128 in Methods in Molecular Biology, Vol. 7: Gene Transfer and Expression Protocols, Murray, E. (ed.), Humana Press, Clifton. N.J. (1991); Miller, N., et al., FASEB Journal 9: 190-199, 1995; Schreier, H. Pharmaceutica Acta Helvetiae 68: 145-159, 1994; Schneider and French, Circulation 88:1937-1942, 1993; Curiel D.T., et al., Human Gene Therapy 3: 147-154, 1992; Graham, F.L., et al., WO 95/00655 (5 January 1995); Falck-Pedersen, E.S., WO 95/16772 (22 June 1995); Denefle. P. et al., WO 95/23867 (8 September 1995); Haddada, H. et al., WO 94/26914 (24 November 1994); Perricaudet, M. et al., WO 95/02697 (26 January 1995); Zhang, W., et al., WO 95/25071 (12 October 1995). A variety of adenovirus plasmids are also available from commercial sources, including, e.g., Microbix Biosystems of Toronto, Ontario (see, e.g., Microbix Product Information Sheet: Plasmids for Adenovirus Vector Construction, 1996). See also, the papers by Vile et al., Nature Biotechnology, 15: 840-841, 1997, Feng et al., Nature Biotechnology, 15: 866-870, 1997, describing the construction and use of adenoviral/retroviral chimeric vectors that can be employed for gene delivery.

Additional references describing AAV vectors which could be used in the methods of the present invention include the following: Carter, B., Handbook of Parvoviruses, vol. I, pp. 169-228, 1990; Berns, Virology, pp. 1743-1764 (Raven Press 1990); Carter, B., Curr. Opin. Biotechnol., 3: 533-539, 1992; Muzyczka, N., Current Topics in Microbiology and Immunology, 158: 92-129, 1992; Flotte, T.R., et al., Am. J. Respir. Cell Mol. Biol. 7:349-356, 1992; Chatterjee et al., Ann. NY Acad. Sci., 770: 79-90, 1995; Flotte, T.R., et al., WO 95/13365 (18 May 1995); Trempe, J.P., et al., WO 95/13392 (18 May 1995); Kotin, R., Human Gene Therapy, 5: 793-801, 1994; Flotte, T.R., et al., Gene Therapy 2:357-362, 1995; Allen, J.M., WO 96/17947 (13 June 1996); and Du et al., Gene Therapy 3: 254-261, 1996.

Additional references describing non-viral vectors which could be used in the methods of the present invention include the following: Ledley, FD, Human Gene Therapy 6: 1129-1144, 1995; Miller, N., et al., FASEB Journal 9: 190-199, 1995; Chonn. A., et al., Curr. Opin. in Biotech. 6: 698-708, 1995: Schofield. J.P., et al., British Med. Bull. 51: 56-71, 1995: Brigham. K.L., et al., J. Liposome Res. 3: 31-49, 1993; Brigham, K.L.. WO 91/06309 (16 May 1991); Felgner, P.L., et al., WO 91/17424 (14 November 1991); Solodin et al., Biochemistry 34: 13537-13544, 1995; WO 93/19768 (14 October 1993); Debs et al., WO 93/25673; Feigner, P.L., et al., U.S. Patent 5,264,618 (November 23, 1993); Epand. R.M., et al., U.S. Patent 5,283,185 (February I, 1994); Gebeyehu et al., U.S. Patent 5,334,761 (August 2, 1994); Felgner, P.L., et al., U.S. Patent 5,459,127 (October 17, 1995); Overell, R.W., et al., WO 95/28494 (26 October 1995); Jessee, WO 95/02698 (26 January 1995); Haces and Ciccarone. WO 95/17373 (29 June 1995); Lin et al.. WO 96/01840 (25 January 1996).

### Tissue Specific Promoters

The present invention also contemplates the use of cell targeting not only by delivery of the transgene into the coronary artery, or femoral artery, for example, but also the use of tissue-specific promoters. By fusing, for example, tissue-specific transcriptional control sequences of left ventricular myosin light chain-2 (MLC_{2V}) or myosin heavy chain (MHC) to a transgene such as the FGF-5 gene within the adenoviral construct, transgene expression is limited to ventricular cardiac myocytes. The efficacy of gene expression and degree of specificity provided by MLC_{2V} and MHC promoters with lacZ have been determined, using the recombinant adenoviral system of the present invention. Cardiac-specific expression has been reported previously by Lee, et al. *(J Biol* Chem 267:15875-15885,1992). The MLC_{2V} promoter is comprised of 250 bp, and fits easily within the adenoviral-5 packaging constraints. The myosin heavy chain promoter, known to be a vigorous promoter of transcription, provides a reasonable alternative cardiac-specific promoter and is comprised of less than 300 bp. Other promoters, such as the troponin-C promoter, while highly efficacious and sufficiently small, does less than lack adequate tissue specificity. By using the MLC_{2V} or MHC promoters and delivering the transgene *in vivo,* it is believed that the cardiac myocyte alone (that is without concomitant expression in endothelial cells, smooth muscle cells, and fibroblasts within the heart) will provide adequate expression of an angiogenic protein such as FGF-5 to promote angiogenesis. Limiting expression to the cardiac myocyte also has advantages regarding the utility of gene transfer for the treatment of clinical myocardial ischemia. By limiting expression to the heart, one avoids the potentially harmful effect of angiogenesis in non-cardiac tissues such as the retina. In addition, of the cells in the heart, the myocyte would likely provide the longest transgene expression since the cells do not undergo rapid turnover; expression would not therefore be decreased by cell division and death as would occur with endothelial cells. Endothelial-specific promoters are already available for this purpose (Lee. et al., *J Biol Chem* 265:10446-10450. 1990).

In the present invention, with regard to the treatment of heart disease, targeting the heart by intracoronary injection with a high titer of the vector and transfecting all cell types is presently preferred.

### Propagation and Purification of Adenovirus Vectors

Successful recombinant vectors can be plaque purified according to standard methods. The resulting viral vectors are propagated on 293 cells which provide ElA and El B functions in trans to titers in the preferred 10¹⁰-10¹² viral particles/ml range. Cells can be infected at 80% confluence and harvested 48 hours later. After 3 freeze-thaw cycles the cellular debris is pelleted by centrifugation and the virus purified by CsCl gradient ultracentrifugation (double CsCl gradient ultracentrifugation is preferred). Prior to *in vivo* injection, the viral stocks are desalted by gel filtration through Sepharose columns such as G25 Sephadex. The product is then filtered through a 30 micron filter, thereby reducing deleterious effects of intracoronary injection of unfiltered virus (life threatening cardiac arrhythmias) and promoting efficient gene transfer. The resulting viral stock has a final viral titer in the range of 10¹¹-10¹² viral particles/ml. The recombinant adenovirus must be highly purified, with no wild-type (potentially replicative) virus. Impure constructs can cause an intense immune response in the host animal. From this point of view, propagation and purification may be conducted to exclude contaminants and wild-type virus by, for example, identifying successful recombinants with PCR using appropriate primers, conducting two rounds of plaque purification, and double CsCl gradient ultracentrifugation. Additionally, we have found that the problems associated with cardiac arrhythmias induced by adenovirus vector injection into patients can be avoided by filtration of the recombinant adenovirus through an appropriately-sized filter prior to intracoronary injection. This strategy also appears to substantially improve gene transfer and expression.

### Delivery of Recombinant Vectors

Vectors of the present invention, including viral vectors as well as non-viral vectors, can be in the form of an injectable preparation containing pharmaceutically acceptable carrier such as saline, for example, as necessary.

A preferred vector is a replication-deficient adenoviral vector as described above. The final titer of such vector in the injectable preparation is preferably in the range of 10⁷-10¹³ viral particles which allows for effective gene transfer. Other pharmaceutical carriers, formulations and dosages are described below. The vector transgene constructs are delivered to the myocardium by infusing one or both coronary arteries (or graft vessel) using standard percutaneous catheter based methods under fluoroscopic guidance, at an amount sufficient for the transgene to be expressed to a degree which allows for highly effective therapy.

The injection should be made deeply into the lumen (about 1 cm within the arterial lumen) of the coronary arteries (or graft vessel), and preferably be made in both coronary arteries, as the growth of collateral blood vessels is highly variable within individual patients. Thus, injection into one coronary artery or graft artery is contemplated; however, injection into both the right and left coronary circulation is preferred. Delivery by three injections, one each into the left anterior descending (LAD) and left circumflex (LCx) coronary artery, and one into the right coronary artery, is particularly preferred. By injecting the material directly into the lumen of the coronary artery by coronary catheters (preferably in combination with a vasoactive agent, for example histamine or a histamine agonist or a VEGF protein where enhanced gene therapy is desired), it is possible to target the gene rather effectively, and to minimize loss of the recombinant vectors to the proximal aorta during injection. We have found that gene expression when delivered in this manner does not occur in hepatocytes and viral RNA cannot be found in the urine at any time after intracoronary injection. Any variety of coronary catheter, or a Stack perfusion catheter, for example, can be used in the present invention. In addition, other techniques known to those having ordinary skill in the art can be used for transfer of genes to the arterial wall.

Gene delivery to other organs or tissues via intra-arterial infusion into an artery supplying the target tissue can be carried out in an analogous manner, preferably in combination with infusion of a vasoactive agent, for example histamine or a histamine agonist or a vascular endothelial growth factor (VEGF) protein, to enhance gene delivery as described and illustrated herein.

By way of illustration, for the treatment of peripheral vascular disease (which is characterized by insufficient blood supply to the legs), recombinant vectors expressing an angiogenic peptide or protein can be delivered by a catheter inserted into the proximal portion of the femoral artery or arteries, preferably in combination with a vasoactive agent, for example histamine or a histamine agonist, thereby effecting gene transfer into the cells of the skeletal muscles receiving blood flow from the femoral arteries. This will provide an angiogenic stimulus that will result in angiogenesis and a concomitant increase in blood flow in the treated skeletal muscle of the legs.

A vasoactive agent, as used herein, refers to a natural or synthetic substance that induces increased vascular permeability and enhances transfer of macromolecules such as gene delivery vectors across capillary walls. By augmenting vascular permeability to macromolecules or otherwise facilitating the transfer of macromolecules into the capillary bed perfused by an artery, vasoactive agents can enhance delivery of these vectors to the targeted sites and thus effectively enhance overall expression of the transgene in the target tissue. In the illustrative Examples below (see, e.g., Example 5), histamine was employed as a vasoactive agent and was found to substantially enhance delivery of a vector to an infused site such as the myocardium. Histamine derivatives (and agonists such as those that interact with the histamine H, receptor) which can be employed include, for example, 2-methylhistamine, 2-pyridylethylamine, betahistine, and 2 thiazolylethylamine. These and additional histamine agonists are described, for example, in Garrison JC., Goodman and Gilman's The Pharmacological Basis of Therapeutics (8th Ed: Gilman AG, Rall TW, Nies AS, Taylor P, eds) Pergamon Press. 1990, pp 575-582.

In addition to histamine and histamine agonists which can be employed as vasoactive agents, vascular endothelial growth factors (VEGFs) and VEGF agonists (as described above and in the cited references) can also induce increased vascular permeability and can therefore be used as a vasoactive agent to enhance gene delivery in the context of the compositions and methods described herein. As with histamine, the VEGF is preferably infused into a blood vessel supplying the target site over several minutes prior to infusion of vector. Human VEGFs have been described, for example, by Tischer et al. J. Biol. Chem. 206:11947-11954, 1991, and references therein; and by Muhlhauser et al., Cir. Res. 77:1077-1086, 1995.

Vasoactive agents can be introduced coincident with administration of vector, but are preferably introduced to the target site (e.g. by pre-infusion) within several minutes prior to the introduction of the vector so that the vasoactive agent is able to elicit a response in the infused cells prior to exposure of the cells to the vector. Without wishing to be bound by theory, it is believed that administration of such a vasoactive agent can enhance transcapillary vesicular transport across the capillary endothelial cell wall, thereby facilitating the transfer of macromolecular complexes such as viral vectors.

### Animal Model of Myocardial Ischemia

Important prerequisites for successful studies on gene therapy are (a) constitution of an animal model which is applicable to clinical myocardial ischemia which can provide useful data regarding mechanisms for angiogenesis in the setting of myocardial ischemia, and (b) accurate evaluation of the effects of gene transfer. From this point of view, none of the prior art is satisfactory. We have made use of a porcine model of myocardial ischemia that mimics clinical coronary artery disease. Placement of an ameroid constrictor around the left circumflex (LCx) coronary artery results in gradual complete closure (within 7 days of placement) with minimal infarction (1% of the left ventricle, 4 ± 1% of the LCx bed) (Roth, et al. *Circulation* 82:1778, 1990, Roth, et al. *Am J Physiol* 235:H1279, 1987, White, et al. *Circ Res* 71:1490, 1992, Hammond, et al. *Cardiol* 23:475, 1994, and Hammond, et al. *J Clin Invest* 92:2644, 1993). Myocardial function and blood flow are normal at rest in the region previously perfused by the occluded artery (referred to as the ischemic region), due to collateral vessel development, but blood flow reserve is insufficient to prevent ischemia when myocardial oxygen demands increase. Thus, the LCx bed is subject to episodic ischemia, analogous to clinical *angina* pectoris. Collateral vessel development and flow-function relationships are stable within 21 days of ameroid placement, and remain unchanged for four months (Roth, et al. *Circulation* 82:1778, 1990, Roth, et al. *Am J Physiol* 235:H1279, 1987, White, et al. *Circ Res* 71:1490, 1992). It has been documented by telemetry that animals have period ischemic dysfunction in the bed at risk throughout the day, related to abrupt increases in heart rate during feeding, interruptions by personnel, etc. (unpublished data). Thus, the model has a bed with stable but inadequate collateral vessels, and is subject to periodic ischemia. Another distinct advantage of the model is that there is a normally perfused and functioning region (the LAD bed) adjacent to an abnormally perfused and functioning region (the LCx bed), thereby offering a control bed within each animal.

Myocardial contrast echocardiography was used to estimate regional myocardial perfusion. The contrast material is composed of microaggregates of galactose and increases the echogenicity (whiteness) of the image. The microaggregates distribute into the coronary arteries and myocardial walls in a manner that is proportional to blood flow (Skyba, et al. *Circulation* 90:1513-1521, 1994). It has been shown that peak intensity of contrast is closely correlated with myocardial blood flow as measured by microspheres (Skyba, et al. *Circulation* 90:1513-1521, 1994). To document that the echocardiographic images employed in the present invention were accurately identifying the LCx bed, and that myocardial contrast echocardiography could be used to evaluate myocardial blood flow, a hydraulic cuff occluder was placed around the proximal LCx adjacent to the ameroid.

In the present study, when animals were sacrificed, the hearts were perfusion-fixed (glutaraldehyde, physiological pressures, *in situ)* in order to quantitate capillary growth by microscopy. PCR was used to detect angiogenic protein DNA and mRNA in myocardium from animals that had received gene transfer. In addition, as described below, two weeks after gene transfer, myocardial samples from all five lacZ-infected animals show substantial β-galactosidase activity on histological inspection. Finally, using a polygonal antibody to an angiogenic protein, angiogenic protein expression in cells and myocardium from animals that had received gene transfer was demonstrated.

The strategy for therapeutic studies included the timing of transgene delivery, the route of administration of the transgene, and choice of the angiogenic gene. In the ameroid model of myocardial ischemia, gene transfer was performed after stable but insufficient collateral vessels had developed. Previous studies using the ameroid model involved delivery of angiogenic peptides during the closure of the ameroid, prior to the development of ischemia and collateral vessels. However, this strategy was not employed for several reasons. First, previous studies are not suitable for closely duplicating the conditions that would be present in the treatment of clinical myocardial ischemia in which gene transfer would be given in the setting of ongoing myocardial ischemia; previous studies are analogous to providing the peptide in anticipation of ischemia, and are therefore less relevant. Second, it was presumed; based upon previous studies in cell culture, that an ischemic stimulus in conjunction with the peptide would be the optimal milieu for the stimulation of angiogenesis. This could optimally be achieved by delivery of the transgene at a time when myocardial ischemia was already present. Linked to these decisions was the selection of the method to achieve transgene delivery. The constraint that the technique should be applicable for the subsequent treatment of patients with coronary disease, made several approaches untenable (continuous infusion of a peptide into the coronary artery, direct plasmid injection into the heart, coating the heart with a resin containing the peptide to provide long-term slow release). Finally, the pig model provided an excellent means to follow regional blood flow and function before and after gene delivery. The use of control animals that received the same recombinant adenovirus construct but with a reporter gene provided a control for these studies. Those skilled in the art will understand that the results described below in pigs are predictive of results in humans. The pig has a native coronary circulation very similar of that of humans, including the absence of native coronary collateral vessels.

### Therapeutic Applications

The vectors of the present invention such as the replication-deficient adenovirus allow for highly efficient gene transfer *in vivo* without cytopathic effect or inflammation in the areas of gene expression. Based on these results, described further in the below Examples, it is seen that a high enough degree of *in vivo* gene transfer to effect *in vivo* functional changes is achieved. In the case of treating a heart disease, the gene transfer of an angiogenic protein by intracoronary injection will promote angiogenesis and enhance cardiac function. Thus, treatment of cardiac ischemia can be conducted after observation of initial ischemic episodes. In addition, after gene transfer, capillary number, blood flow and function will increase in the ischemic region. Application of these techniques clinically will be of great utility, especially initially in those with inoperative coronary artery disease and disabling *angina pectoris.* The data of the present invention demonstrate that gene transfer of a recombinant adenovirus expressing fibroblast growth factor (e.g., FGF-5) is effective in substantially reducing myocardial ischemia. Angiogenic proteins and transgenes can also be employed in the compositions and methods of treatment described in co-pending application U.S. Serial No. 08/852,779, filed May 6, 1997, incorporated by reference herein. As described herein, a vasoactive agent, for example histamine or a histamine agonist or a VEGF protein, can be employed in conjunction with these methods and compositions in order to further enhance gene delivery at the site being targeted by intravascular injection.

As noted above, β-adrenergic signaling proteins (β-ASPs), for example β-adrenergic receptors (β-ARs), G-protein receptor kinase inhibitors (GRK inhibitors) and adenylylcyclases (ACs), can also be employed to enhance cardiac function as described and illustrated in detail in co-pending applications U.S. Serial No. 08/924,757, filed 05 September 1997 (based on U.S. 60/048,933 filed 16 June 1997 and U.S. 08/708,661 filed 05 September 1996), as well as PCT/US97/15610 filed 05 September 1997, and U.S. continuing case Serial No. 08/_, filed 16 January 1998, each of which is incorporated by reference herein. As described herein, a vasoactive agent, for example histamine or a histamine agonist or a VEGF protein, can be employed in conjunction with these methods and compositions in order to further enhance gene delivery at the site being targeted by intravascular injection.

Compositions or products of the invention may conveniently be provided in the form of formulations suitable for intracoronary administration. A suitable administration format may best be determined by a medical practitioner for each patient individually. Suitable pharmaceutically acceptable carriers and their formulation are described in standard formulations treatises, e.g., *Remington's Pharmaceuticals Sciences* by E.W. *Martin. See also* Wang, Y.J. and Hanson, M.A. "Parental Formulations of Proteins and Peptides: Stability and Stabilizers," *Journals of Parental Sciences and Technology,* Technical Report No. 10, Supp. 42:2S (1988). Vectors of the present invention should preferably be formulated in solution at neutral pH, for example, about pH 6.5 to about pH 8.5, more preferably from about pH 7 to 8, with an excipient to bring the solution to about isotonicity, for example, 4.5% mannitol or 0.9% sodium chloride, pH buffered with art-known buffer solutions, such as sodium phosphate, that are generally regarded as safe, together with an accepted preservative such as metacresol 0.1% to 0.75%, more preferably from 0.15% to 0.4% metacresol. The desired isotonicity may be accomplished using sodium chloride or other pharmaceutically acceptable agents such as dextrose, boric acid, sodium tartrate, propylene glycol, polyols (such as mannitol and sorbitol), or other inorganic or organic solutes. Sodium chloride is preferred particularly for buffers containing sodium ions. If desired, solutions of the above compositions may also be prepared to enhance shelf life and stability. The therapeutically useful compositions of the invention are prepared by mixing the ingredients following generally accepted procedures. For example, the selected components may be mixed to produce a concentrated mixture which may then be adjusted to the final concentration and viscosity by the addition of water and/or a buffer to control pH or an additional solute to control tonicity.

For use by the physician, the compositions will be provided in dosage form containing an amount of a vector of the invention which will be effective in one or multiple doses to induce angiogenesis at a selected level. As will be recognized by those in the field, an effective amount of therapeutic agent will vary with many factors including the age and weight of the patient, the patient's physical condition, and the level of angiogenesis to be obtained, and other factors.

The effective dose of a preferred compound of this invention such as the replication-deficient adenovirus, will typically be in the range of at least about 10⁷ viral particles, preferably about 10⁹ viral particles, and more preferably about 10¹¹ viral particles. The number of viral particles may, but preferably does not exceed 10¹³. As noted, the exact dose to be administered is determined by the attending clinician, but is preferably in 1 ml phosphate buffered saline.

The presently preferred mode of administration in the case of heart disease is by intracoronary injection to one or both coronary arteries (or to one or more saphenous vein or internal mammary artery grafts) using an appropriate coronary catheter. As described herein, injection into both the right and left coronary circulation is preferred. Delivery by three injections, one each into the left anterior descending (LAD) and left circumflex (LCx) coronary artery, and one into the right coronary artery, is particularly preferred. Although it is of course convenient to perform the two left coronary injections in series, these can be performed prior to or following injection into the right coronary artery. In situations in which the right coronary artery is not readily accessible (as a result of occlusion without bridging collaterals for example or because it is too small for selective injection), the entire dose of vector can be delivered via injection into the left coronary circulation (preferably LAD and LCx as noted above). Each injection is generally performed over a period of one to several minutes, typically about one and a half minutes. The presently preferred mode of administration in the case of peripheral vascular disease is by injection into the proximal portion of the femoral artery or arteries using an appropriate arterial catheter.

As described herein, vasoactive active agents can be infused at the target site coincident with or prior to the delivery of vector by, for example, a catheter to promote gene transfer. Use of such agents resulted in an enhancement in gene delivery efficiency that was evidenced by an increase in the percentage of cells in the targeted site that express the transgene. With regard to physiological and pharmacological effects of histamine and other vasoactive agents, see, e.g., Altura BM and Halevy S. Cardiovascular actions of histamine. In: Histamine II and Anti-Histaminics: Chemistry, Metabolism and Physiological and Pharmacological Actions (Rocha e Silva M, ed) Handbuch der Experimentellen Pharmakologie, Vol 18, Part 2. Springer Verlag, Berlin, 1978, pp 1-39. Histamine derivatives (and agonists such as those that interact with the histamine H, receptor) which can be employed include, for example, 2-methylhistamine, 2-pyridylethylamine, betahistine, and 2 thiazolylethylamine. These and additional histamine agonists are described, for example, in Garrison JC., Goodman and Gilman's The Pharmacological Basis of Therapeutics (8th Ed: Gilman AG, Rall TW, Nies AS, Taylor P, eds) Pergamon Press, 1990, pp 575-582.

To assist in understanding the present invention, the following Examples are provided which describe the results of a series of experiments. The experiments relating to this invention should not, of course, be construed as specifically limiting the invention and such variations of the invention, now know or later developed, which would be within the purview of one skilled in the art are considered to fall within the scope of the invention as described herein and hereinafter claimed.

### EXAMPLE 1: ADENOVIRAL CONSTRUCTS

A helper independent replication deficient human adenovirus 5 system was used. The genes of interest were lacZ and FGF-5. The full length cDNA for human FGF-5 was released from plasmid pLTRI22E (Zhen, et al. *Mol Cell Biol* 8:3487. 1988) as a 1.1 kb EcoR1 fragment which includes 981 bp of the open reading frame of the gene, and cloned into the polylinker of plasmid ACCMVPLPA which contains the CMV promoter and SV40 polyadenylation signal flanked by partial adenoviral sequences from which the ElA and EIB genes (essential for viral replication) had been deleted. This plasmid was cotransfected (lipofection) into 293 cells with plasmid JM 17 which contained the entire human adenoviral 5 genome with an additional 4.3 kb insert making pJM17 too large to be encapsulated. Homologous rescue recombination resulted in adenoviral vectors containing the transgene in the absence of ElA/ElB sequences. Although these recombinants were nonreplicative in mammalian cells, they could propagate in 293 cells which had been transformed with E1A/E1B and provided these essential gene products in trans. Transfected cells were monitored for evidence of cytopathic effect which usually occurred 10-14 days after transfection. To identify successful recombinants, cell supernatant from plates showing a cytopathic effect was treated with proteinase K (50 mg/ml with 0.5% sodium dodecyl sulfate and 20 mM EDTA) at 56°C for 60 minutes, phenol/chloroform extracted and ethanol precipitated. Successful recombinants were then identified with PCR using primers *(Biotechniques* 15:868-872, 1993) complementary to the CMV promoter and SV40 polyadenylation sequences to amplify the insert (the expected 1.1 kb fragment), and primers *(Biotechniques* 15:868-872, 1993) designed to concomitantly amplify adenoviral sequences. Successful recombinants then underwent two rounds of plaque purification. Viral stocks were propagated in 293 cells to titers ranging between 10¹⁰ and 10¹² viral particles, and were purified by double CsCl gradient centrifugation prior to use. Recombinant adenoviruses encoding β-galactosidase, or FGF-5 were constructed using full length cDNAs. The system used to generate recombinant adenoviruses imposed a packing limit of 5 kb for transgene inserts. The genes proposed, driven by the CMV promoter and with the SV40 polyadenylation sequences were less than 4 kb, well within the packaging constraints. Recombinant vectors were plaque purified by standard procedures. The resulting viral vectors were propagated on 293 cells to titers in the 10¹⁰-10¹² viral particles range. Cells were infected at 80% confluence and harvested at 36-48 hours. After freeze-thaw cycles the cellular debris was pelleted by standard centrifugation and the virus further purified by double CsCl gradient ultracentrifugation (discontinuous 1.33/1.45 CsCl gradient: cesium prepared in 5 mM Tris, 1 mM EDTA (pH 7.8); 90,000 x g (2 hr), 105,000 x g (18 hr)). Prior to *in vivo* injection, the viral stocks were desalted by gel filtration through Sepharose columns such as G25 Sephadex. The resulting viral stock had a final viral titer in the 10¹¹-10¹² viral particles range. The adenoviral construct was highly purified, with no wild-type (potentially replicative) virus.

### EXAMPLE 2: ADULT RAT CARDIOMYOCYTES IN CELL CULTURE

Adult rat cardiomyocytes were prepared by Langendorf perfusion with a collagenase containing perfusate according to standard methods. Rod shaped cells were cultured on laminin coated plates and at 24 hours were infected with the β-galactosidase-encoding adenovirus obtained in the above Example 1 at a multiplicity of infection of 1:1. After a further 36 hour period the cells were fixed with glutaraldehyde and incubated with X-gal. Consistently 70-90% of adult myocytes expressed the β-galactosidase transgene after infection with the recombinant adenovirus. At a multiplicity of infection of 1-2:1 there was no cytotoxicity observed.

### EXAMPLE 3: PORCINE MYOCARDIUM IN VIVO

The β-galactosidase-encoding adenoviral vector obtained in Example 1 was propagated in permissive 293 cells and purified by CsCl gradient ultracentrifugation with a final viral titer of 1.5 x 10¹⁰ viral particles, based on the procedures of Example 1. An anesthetized, ventilated 40 kg pig underwent thoracotomy. A 26 gauge butterfly needle was inserted into the mid left anterior descending (LAD) coronary artery and the vector (1.5 x 10¹⁰ viral particles) was injected in a 2 ml volume. The chest was closed and the animal allowed to recover. On the fourth post-injection day the animal was killed. The heart fixed with glutaraldehyde, sectioned and incubated with X-gal for 16.5 hours. After imbedding and sectioning the tissue was counterstained with eosin.

Microscopic analysis of tissue sections (transmural sections of LAD bed 96 hours after intracoronary injection of adenovirus containing lacZ) revealed a significant magnitude of gene transfer observed in the LAD coronary bed with many tissue sections demonstrating greater than 50-60% of the cells staining positively for β-galactosidase. Areas of the myocardium remote from the LAD circulatory bed did not demonstrate X-gal staining and served as a negative control, while diffuse expression of a gene was observed in myocytes and in endothelial cells. The majority of myocytes showed β-galactosidase activity (blue stain), and, in subsequent studies using closed-chest intracoronary injection, similar activity was present 14 days after gene transfer (n=8). There was no evidence of inflammation or necrosis in areas of gene expression

### EXAMPLE 4: PORCINE ISCHEMIA MODEL

Animals included 18 domestic pigs (30-40 kg). A left thoracotomy was performed under sterile conditions for instrumentation. (Hammond, et al. *J Clin Invest* 92:2644-2652, and Roth, et al. *J Clin Invest* 91:939-949, 1993). Catheters were placed in the left atrium and aorta, providing a means to measure regional blood flow, and to monitor pressures. Wires were sutured on the left atrium to permit ECG recording and atrial pacing. Finally, an ameroid was placed around the proximal LCx. After a stable degree of ischemia had developed, the treatment group (n=11) received an adenoviral construct that included FGF-5 (an angiogenic gene), driven by a CMV promoter. Control animals (n=7) received gene transfer with an adenoviral construct that included a reporter gene, lacZ, driven by a CMV promoter.

Studies were initiated 35 ± 3 days after ameroid placement, at a time when collateral vessel development and pacing-induced dysfunction were stable (Roth, et al. *Am J Physiol* 253:H1279-1288, 1987, and Roth, et al. *Circulation* 82:1778-1789). Conscious animals were suspended in a sling and pressures from the LV, LA and aorta, and electrocardiogram were recorded in digital format on-line (at rest and during atrial pacing at 200 bpm). Two dimensional and M-mode images were obtained using a Hewlett Packard ultrasound imaging system. Images were obtained from a right parasternal approach at the mid-papillary muscle level and recorded on VHS tape. Images were recorded with animals in a basal state and again during right atrial pacing (HR=200 bpm). These studies were performed one day prior to gene transfer and repeated 14 ± 1 days later. Rate-pressure products and left atrial pressures were similar in both groups before and after gene transfer, indicating similar myocardial oxygen demands and loading conditions. Echocardiographic measurements were made using standardized criteria (Sahn, et al. *Circulation* 58:1072, 1978). End-diastolic wall thickness (EDWTh) and end-systolic wall thickness (ESWTh) were measured from 5 continuous beats and averaged. Percent wall thickening (%WTh) was calculated [(EDWTh-ESWTh)/EDWTh] X 100. Data were analyzed without knowledge of which gene the animals had received. To demonstrate reproducibility of echocardiographic measurements, animals (n=5) were imaged on two consecutive days, showing high correlation (r²=0.90; p=0.005).

35 ± 3 days after ameroid placement, well after ameroid closure, but before gene transfer, contrast echocardiographic studies were performed using the contrast material (Levovist) which was injected into the left atrium during atrial pacing (200 bpm). Studies were repeated 14 ± 1 days after gene transfer. Peak contrast intensity was measured from the video images using a computer-based video analysis program (Color Vue II, Nova Microsonics, Indianapolis, Indiana), that provided an objective measure of video intensity. The contrast studies were analyzed without knowledge of which gene the animals had received.

At completion of the study, animals were anesthetized and midline thoracotomy performed. The brachycephalic artery was isolated, a cannula inserted, and other great vessels ligated. The animals received intravenous heparin (10,000 IU) and papaverine (60 mg). Potassium chloride was given to induce diastolic cardiac arrest, and the aorta cross-clamped. Saline was delivered through the brachycephalic artery cannula (120 mmHg pressure), thereby perfusing the coronary arteries. Glutaraldehyde solution (6.25%, 0.1 M cacodylate buffer) was perfused (120 mmHg pressure) until the heart was well fixed (10-15 min). The heart was then removed, the beds identified using color-coded dyes injected anterograde through the left anterior descending (LAD), left circumflex (LCx), and right coronary arteries. The ameroid was examined to confirm closure. Samples taken from the normally perfused and ischemic regions were divided into thirds and the endocardial and epicardial thirds were plastic-imbedded. Microscopic analysis to quantitate capillary number was conducted as previously described (Mathieu-Costello, et al. *Am J Physiol* 369:H204, 1990). Four 1 µm thick transverse sections were taken from each subsample (endocardium and epicardium of each region) and pointcounting was used to determine capillary number per fiber number ratio at 400X magnification. Twenty to twenty-five high power fields were counted per subsample. Within each region, capillary number to fiber number rations were similar in endocardium and epicardium so the 40-50 field per region were averaged to provide the transmural capillary to fiber number ratio.

To establish that improved regional function and blood flow resulted from transgene expression, PCR and PT-PCR were used to detect transgenic FGF-5 DNA and mRNA in myocardium from animals that had received FGF-5 gene transfer. Using a sense primer to the CMV promoter [GCAGAGCTCGTTTAGTGAAC] (SEQ ID NO.:1) and an antisense primer to the internal FGF-5 sequence [GAAAATGGGTAGAGATATGCT] (SEQ ID NO.:2), PCR amplified the expected 500 bp fragment. Using a sense primer to the beginning of the FGF-5 sequence [ATGAGCTTGTCCTTCCTCCTC] (SEQ ID NO.:3) and an antisense primer to the internal FGF-5 sequence [GAAAATGGGTAGAGATATGCT] (SEQ ID NO.:2), RT-PCR amplified the expected 400 bp fragment.

Finally, using a polygonal antibody directed against FGF-5 (Kitaoka, et al. *Science* 35:3189, 1994), FGF-5 protein expression was demonstrated 48 hours as well as 14 ± 1 days after FGF-5 gene transfer in cells and myocardium from animals that had received gene transfer with FGF-5.

The helper independent replication deficient human adenovirus 5 system constructed in Example 1 was used to prepare transgene containing vectors. The genes of interest were lacZ and FGF-5. The material injected *in vivo* was highly purified and contained no wild-type (replication competent) adenovirus. Thus adenoviral infection and inflammatory infiltration in the heart were minimized. By injecting the material directly into the lumen of the coronary artery by coronary catheters, it was possible to target the gene effectively. When delivered in this manner there was no transgene expression in hepatocytes, and viral RNA could not be found in the urine at any time after intracoronary injection.

Injection of the construct (4.0 ml containing about 10¹¹ viral particles of adenovirus) was made by injecting 2.0 ml into both the left and right coronary arteries (collateral flow to the LCx bed appeared to come from both vessels). Animals were anesthetized, and arterial access acquired via the right carotid by cut-down; a 5F Cordis sheath was placed. A 5F Multipurpose (A2) coronary catheter was used to engage the coronary arteries. Closure of the LCx ameroid was confirmed by contrast injection into the left main coronary artery. The catheter tip was then placed 1 cm within the arterial lumen so that minimal material would be lost to the proximal aorta during injection. This procedure was carried out for each of the pigs.

Once gene transfer was performed, three strategies were used to establish successful, incorporation and expression of the gene. (1) Some constructs included a reporter gene (lacZ); (2) myocardium from the relevant beds was sampled, and immunoblotting was performed to quantitate the presence of FGF-5; and (3) PCR was used to detect FGF-5 mRNA and DNA.

The regional contractile function data in Figure 2 shows that pigs receiving lacZ showed a similar degree of pacing-induced dysfunction in the ischemic region before and 14 ± 1 days after gene transfer. In contrast, pigs receiving FGF-5 gene transfer showed a 2.6 fold increase in wall thickening in the ischemic region during pacing (p=0.0001). These data demonstrate that FGF-5 gene transfer in accordance with the invention was associated with improved contraction in the ischemic region during pacing. Wall thickening in the normally perfused region (the interventricular septum) was normal during pacing and unaffected by gene transfer (% Wall Thickening: lacZ Group: Pregene, 56 ± 11 %, Post-gene, 51 ± 9%; FGF-5 Group: Pre-gene, 63 ± 7%, Postgene, 58 ± 5%; no differences, two-way analysis of variance). The data from the separate determinations were highly reproducible (lateral wall thickening: r²=0.90; p=0.005). The percent decrease in function measured by transthoracic echocardiography was very similar to the percentage decrease measured by sonomicrometry during atrial pacing in the same model (Hammond, et al. *J Clin Invest* 92:2644, 1993), documenting the accuracy of echocardiography for the evaluation of ischemic dysfunction. Bars in Figure 2 represent mean values, error bars denote 1 SE. Figures 4A-4C are diagrams corresponding to myocardial contrast echocardiographs. Figure 4A illustrates acute LCx occlusion in a normal pig, in which no flow is indicated in LCx bed (black) while septum (IVS) enhances (white), confirming that the image accurately identified the LCx bed and that reduced blood flow was associated with reduced contrast enhancement. Figure 4B illustrates the difference in contrast enhancement between IVS and LCx bed 14 days after gene transfer with lacZ, indicating different blood flows in two regions during atrial pacing (200 bpm). In Figure 4C, contrast enhancement appears equivalent in IVS and LCx bed 14 days after gene transfer with FGF-5, indicating similar blood flows in the two regions during atrial pacing.

Figure 3 summarizes computer analysis of video intensity in the two regions from all animals. In Figure 3, data were expressed as the ratio of the peak video intensity (a correlate of myocardial blood flow) in the ischemic region (LCx bed) divided by the peak video intensity in the interventricular septum (IVS, a region receiving normal blood flow through the unoccluded left anterior descending coronary artery). Equal flows in the two regions would yield a ratio of 1.0. The ratio, prior to gene transfer, averaged 0.5, indicates substantially less flow in the LCx bed than in the septum. Figure 3 shows that animals receiving lacZ gene transfer had a persistent blood flow deficit in the ischemic region. Animals receiving FGF-5 gene transfer showed homogeneous contrast enhancement in the two regions, indicating a 2-fold increase in myocardial blood flow improved flow in the ischemic region (p=0.0018, two-way analysis of variance). Bars represent mean values, error bars denote 1 SE.

The bar graph in Figure 5 summarizes the microscopic analysis data, showing increased capillary number to fiber number ratio in the ischemic and nonischemic regions of animals that received gene transfer with FGF-5 when compared to the same regions of the hearts of animals that had received gene transfer with lacZ. Bars represent mean values from 5-6 animals in each group. Error bars denote 1 SE, p value from 2-way analysis of variance for gene effect. The analysis was performed without knowledge of treatment group.

Electropherograms upon the PCR amplification confirmed the presence of JM17-CMV-FGF-5 DNA (the expected 500 bp fragment) in the LAD and LCx beds of three pigs 14 days after gene transfer with FGF-5. Electropherogram upon the RT-PCR amplification confirmed the presence of cardiac FGF-5 mRNA (the expected 400 bp fragment) in the LAD and LCx beds 14 days after gene transfer with FGF-5, but not with lacZ. In addition, two weeks after gene transfer, myocardial samples from all five lacZ-infected animals showed substantial β-galactosidase activity on histological inspection.

Finally, immunoblots of cell medium from cultured fibroblasts with the use of the polygonal antibodies to FGF-5 confirmed protein expression and extracellular secretion 2 days after gene transfer of FGF-5 (n=4 plates), but not after gene transfer of lacZ. Protein expression was also confirmed in myocardial samples 14 ± 1 days after gene transfer of FGF-5 but not after gene transfer of lacZ (n=4).

As described above, adeno-associated viral vectors and other gene delivery vectors known in the art can be employed in an analogous manner.

### EXAMPLE 5: USE OF A VASOACTIVE AGENT TO ENHANCE GENE DELIVERY

In one aspect of the present invention, as described above, the efficiency of gene delivery using a vector such as a viral vector is enhanced by delivering the vector into a blood vessel that is co-infused or pre-infused with a vasoactive agent such as histamine.

In this illustrative example, we have examined the effect of histamine (as an exemplary vasoactive agent) on gene delivery using the aforementioned adenoviral vector that expresses a lacZ reporter gene (as in Example 1). We found that histamine infusion of the coronary artery dramatically increased the gene transfer efficiency.

Briefly, a pig was anesthetized and arterial access obtained via the right carotid artery. A 5 French multipurpose catheter was placed in the right coronary artery (RCA) and inserted 2 cm within the ostia. Approximately three ml of saline was delivered over three minutes, quickly followed by a about 1.5 min infusion of two ml containing 3 x 10¹¹ viral particles ("vp") of adenovirus expressing lacZ. The catheter was then removed from the RCA and placed in the left anterior descending coronary artery (LAD), again inserting the catheter tip 2 cm within the LAD lumen. After insertion, 3 ml of histamine at a concentration of 25 µg histamine per ml was delivered over three minutes, quickly followed by a 2.25 min infusion of three ml solution containing 4.5 x 10¹¹ vp adenoviruses that express lacZ. Typically, histamine infusion was performed within several minutes prior to the delivery of vector. There were no substantial changes in systemic blood pressure, heart rate, or cardiac rhythm during or after histamine infusion. The catheter was removed, the neck closed, and the animal allowed to recover. It should be noted that although the total number of viral particles infused into the LAD bed was about 1.5 times that of the RCA bed (because of the relative sizes of these beds in the pig heart), the increase in total number of viral particles was more than offset by the relative size of the targeted bed since the LAD bed is approximately 2 to 3 fold larger than the RCA bed.

Five days later the animal was sacrificed and the heart perfused with paraformaldehyde. Transmural sections were obtained from the RCA region and the LAD region. Samples were incubated with X-gal solution, the substrate for β-galactosidase, and 16 hr later the material was embedded, sectioned and mounted to glass slides, and stained with eosin.

Microscopic analysis revealed a striking increase in gene transfer efficiency in the LAD bed compared to the RCA bed. In particular, in the absence of histamine treatment, approximately 30% of the cardiac myocytes expressed lacZ after intracoronary infusion. In contrast, almost all myocytes in the LAD region expressed the transgene lacZ, indicating that the effectiveness of gene transfer was increased several fold in the region that had been treated with the vasoactive agent as opposed to the control region. Indeed, the use of a vasoactive agent as illustrated herein resulted in nearly complete gene delivery to the targeted tissue.

## Claims

1. A method for targeted gene expression in vivo, comprising delivering a vector by intra-arterial injection directly into an artery supplying a tissue or organ, wherein the vector comprises a transgene encoding a protein to be expressed in the vascular bed supplied by the artery, and wherein said intra-arterial injection is performed coincident with or after infusion of the artery with a vasoactive agent.

2. The method of Claim 1, wherein said vasoactive agent is infused into the artery at least about 2 minutes prior to the injection of said vector.

3. The method of Claim 1, wherein the vasoactive agent is histamine or a histamine agonist or a vascular endothelial growth factor (VEGF) protein.

4. The method of Claim 1, wherein the vasoactive agent is a histamine or a histamine agonist.

5. The method of Claim 1, wherein said vector is a viral vector.

6. The method of Claim 5, wherein said viral vector is a replication-deficient adenoviral vector.

7. The method of Claim 5, wherein said viral vector is an adeno-associated viral vector.

8. The method of Claim 6, wherein about 10⁷ to about 10¹³ adenovirus vector particles are delivered in the injection.

9. The method of Claim 8, wherein about 10¹¹ adenovirus vector particles are delivered in the injection.

10. The method of Claim 1, wherein said transgene is operably linked to a constitutive promoter.

11. The method of Claim 1, wherein said transgene is operably linked to a tissue-specific promoter.

12. The method of Claim 1, wherein said transgene is operably linked to a ventricular myocyte-specific promoter.

13. The method of Claim 12, wherein said ventricular myocyte-specific promoter has the sequence of ventricular myosin light chain-2.

14. The method of Claim 12, wherein said ventricular myocyte-specific promoter has the sequences of myosin heavy chain promoter.

15. The method of Claim 1, wherein said transgene encodes an angiogenic protein or peptide.

16. The method of any one of Claims 1, or 3 to 7, wherein said transgene encodes an angiogenic protein or peptide, and wherein the vector is delivered to the myocardium of a patient by intracoronary injection(s) directly into one or both coronary arteries, thereby promoting development of coronary collateral vessels in the myocardium of said patient.

17. The method of any one of Claims 1 or 3 to 7, wherein said transgene encodes a β-adrenergic signaling protein, and wherein the vector is delivered to the myocardium of a patient by intracoronary injection directly into one or both coronary arteries.

18. The method of Claim 17, wherein said β-adrenergic signaling protein is selected from the group consisting of a β-adrenergic receptor (β-AR), a G-protein receptor kinase inhibitor (GRK inhibitor) or an adenylylcyclase (AC).

19. The method of Claim 16 or 17, wherein said intracoronary injection is conducted at least about 1cm into the lumens of the left and right coronary arteries.

20. The method of Claim 16 or 17, wherein said intracoronary injection is conducted at least about 1cm into the lumens of a saphenous vein graft or an internal mammary artery graft in addition to a coronary artery.

21. The method of Claim 16 or 17, wherein said method comprises three injections, one each into the left anterior descending (LAD) coronary artery, the left circumflex (LCx) coronary artery and the right coronary artery.

22. The method of any one of Claims 1 or 3 to 7, wherein the vector is introduced to the peripheral vascular system of the patient by intra-femoral artey injection directly into one or both femoral arteries.

23. The method of Claim 22, wherein said vector comprises a transgene coding for an angiogenic protein or peptide, and expresses the transgene in the peripheral vascular system, thereby promoting angiogenesis at a site in the peripheral vascular system.

24. The method of any one of Claims 15, 16 or 23, wherein said angiogenic protein or peptide is selected from the group consisting of aFGF, bFGF, FGF-4, FGF-5, and VEGF.
